# EUROPÄISCHE PATENTSCHRIFT

(11) **EP 2 120 754 B1**
(45) Veröffentlichungstag und Bekanntmachung des Hinweises auf die Patenterteilung: **30.11.2016**
(21) Anmeldenummer: 07856643.7
(22) Anmeldetag: 12.12.2007
(51) Int. Cl.: A61B 17/86

(54) **FIXATIONSSYSTEM FÜR KNOCHEN**
FIXATION SYSTEM FOR BONE
SYSTÈME DE FIXATION POUR OS

(30) Priorität: 20.12.2006 DE 102006060933
(43) Veröffentlichungstag der Anmeldung: 25.11.2009
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2007/010896
(87) Internationale Veröffentlichungsnummer: WO 2008/077491

(56) Entgegenhaltungen:
- EP-A- 0 705 572
- WO-A-2004/112587
- DE-A1- 4 343 117
- US-A- 6 030 162
- US-A1- 2005 059 970

## Beschreibung

Die Erfindung betrifft ein Fixationssystem für Knochen mit einem Verbindungsträger mit wenigstens einem Durchgangsloch, in das eine Knochenschraube eingesetzt wird. Die Knochenschraube weist einen eine Längsachse definierenden Schaft und einen als Verdickung ausgeführten Kopf auf, wobei Schaft und Kopf ein Gewinde tragen.

Knochenschrauben werden in Fixationssystemen für Knochen mit einem Verbindungsträger verwendet, um Knochenstücke miteinander zu verbinden.

Der Verbindungsträger kann eine Knochenplatte sein, wobei der in ein Loch der Knochenplatte eingreifende Schraubenkopf üblicherweise mit der Platte winkelstabil verblockt wird. Eine winkelstabile Verbindung von Knochenschraube und Knochenplatte führt zu einem Stabilitätsgewinn der gesamten Montage. Ist der Schraubenkopf im Plattenloch verblockt, erfolgt die Übertragung der Kräfte und Lasten über die gesamte Länge des im Knochen liegenden Gewindes (Wolter et al., 1999, Universeller Titanfixateur interne - Entwicklungsgeschichte, Prinzip, Mechanik, Implantatgestaltung und operativer Einsatz, Trauma und Berufskrankheit Vol. 1: 307-319). Die einwirkenden Lasten und Kräfte werden proportional der Kontaktfläche der Knochenschraube auf den Knochen verteilt. Die Effektivität des Systems, d.h. die optimal großflächige Verteilung der Lasten und Kräfte, hängt also von der Knochenschrauben-Knochen-Kontaktfläche ab. Untersuchungen haben gezeigt, dass die längsten Schrauben die effektivsten sind. Lange Schrauben sind jedoch nicht immer verwendbar; so sind der Schraubenlänge durch die Anatomie der Knochen Grenzen gesetzt.

Die Merkmale des Oberbegriffs des Anspruchs 1 sind aus EP-A-07 05 572 bekannt.

Aus DE 43 43 117 A1 ist bekannt, dass die Winkelstabilität durch Materialumformung im Bereich der Kontaktflächen von Schraubenkopf und Plattenlochwand erreicht wird.

Bei der Umformung des Materials der Lochwand, welche gemäß DE 198 58 889 A1 beispielsweise als Materiallippe gestaltet sein kann, ist eine Spanbildung möglich, wobei dieser Span aus der Plattenlocheingangsebene heraustreten kann. Dies wird aus Gründen der Verträglichkeit, nicht zuletzt weil es sich bei den Spänen um einen Fremdkörper handelt, der entsprechende Fremdkörper-Reaktionen im umliegenden Gewebe auslösen kann, ebenfalls als ein Nachteil angesehen. Zudem besteht die Gefahr, dass herausragende Späne das benachbarte Gewebe irritieren.

Bei Knochenschrauben der eingangs genannten Art unterscheidet sich üblicherweise das Gewinde des Schraubenkopfes von dem des Schaftes. So weist beispielsweise der Schraubenkopf der winkelstabil verblockbaren Schraube gemäß DE 198 58 889 A1 ein eigenständiges Gewinde mit einer anderen Gewindegeometrie als das des Schraubenschaftes auf. An beide Gewinde werden funktionell unterschiedliche Anforderungen gestellt, was unterschiedliche Gestaltungsformen bedingt.

Das Gewinde im Bereich des Schaftes, das üblicherweise als Knochengewinde bezeichnet wird, dient der Verbindung zum Knochen. Das Knochengewinde ist so gestaltet, dass zwischen den einzelnen Gewindeflanken ein größerer Abstand gegeben ist und gleichzeitig die Dicke des Gewindegrundes reduziert ist. Dadurch ist das Verhältnis zwischen der Knochenmaterialmenge und der Implantatmaterialmenge zu Gunsten der Knochenmaterialmenge im Kontaktbereich verschoben. Da das Implantatmaterial (z.B. Stahl-Titan), d.h. die Knochenschraube, wesentlich höhere Festigkeit als der Knochen aufweist, ist dieses oben beschriebene Verhältnis günstig für die Übertragung von Kräften und Lasten, ohne dass es zur Zerstörung des Knochens im gewöhnlichen Belastungsbereich kommt.

Im Bereich der Verbindung zwischen dem Schraubenkopf und den Platten finden sich andere Verhältnisse. Hier sind vergleichbar feste Materialien in Kontakt, z. B. Reintitan (Platte) mit einer Titanlegierung (Schraubenkopf). Dadurch, dass Reintitan eine weiche und eine Titanlegierung härtere Materialeigenschaften hat, kommt es zu einer Materialumformung im Bereich der Plattenlochwand, wenn ein Gewinde tragender konischer Schraubenkopf aus einer Titanlegierung in das Plattenloch aus Reintitan hineingedreht wird. Das Gewinde am Schraubenkopf ist hier, im Gegensatz zum Knochengewinde der Schraube, so gefertigt, dass die Gewindeflanken kleinere Abstände und geringere Gewindetiefen aufweisen. Bei einer winkelstabilen Verbindung zwischen Knochenplatte und Schraube kommt es zu einer flächenhaften Lastübertragung im Kontaktbereich zwischen Implantat und Knochen.

In bisher angewandten Ausführungen winkelstabiler Platten-Knochenschrauben-Verbindungen endet das Knochengewinde im Halsbereich der Schraube. Der Halsbereich selbst ist häufig dicker als der Kerndurchmesser der Schraube und trägt selbst kein Gewinde. Untersuchungen haben gezeigt, dass der gewindefreie Halsbereich ein besonders kritischer Bereich der Knochenschrauben ist. In diesem Bereich kann es zu Ermüdungsbrüchen bei zu hoher Belastung kommen.

Der Erfindung liegt die Aufgabe zugrunde eine Knochenschraube zu schaffen, die die vorgenannten Nachteile weitestgehend vermeidet.

Die Erfindung löst diese Aufgabe durch ein Fixationssystem für Knochen mit den Merkmalen des Anspruchs 1. Vorteilhafte Ausführungsformen der Erfindung sind in den Unteransprüchen offenbart.

Gemäß Anspruch 1 geht das Knochengewinde des Schaftes unterbrechungsfrei in das Gewinde des aufweitenden Bereiches des Kopfes der Knochenschraube über. Mit anderen Worten, der Schraube fehlt der eigentliche Halsbereich. Weiter erfindungsgemäß weist das Gewinde an dem aufweitenden Bereich des Knochenschraubenkopfes die Gewindesteigung des Knochengewindes auf. Weiter erfindungsgemäß weitet sich der dem Schaft anschließende Kopfbereich in Bezug auf die Längsachse des Schaftes in einen Winkel von 10 bis 28 Grad auf. Bei der Fixierung einer Knochenplatte an einem Knochen mittels der Knochenschraube gemäß des erfindungsgemäßen Fixationssystems tritt der aufweitende Bereich des Kopfes der Knochenschraube durch ein Durchgangsloch der Knochenplatte teilweise hindurch und kommt in Knochengewebe zum Liegen.

Die Erfindung hat erkannt, dass durch die unterbrechungsfreie Weiterführung des Gewindes vom Schaft in den aufweitenden Bereich des Kopfes eine größere Knochenschraube-Knochen-Kontaktfläche geschaffen wird und dadurch eine bessere Verteilung und Übertragung der Lasten und Kräfte über den somit geschaffenen größeren Lastübertragungsbereich erfolgt.

Überraschenderweise hat sich auch herausgestellt, dass das Eintauchen des Schraubenkopfes in das Knochengewebe mit seinem sich an den Schaft unmittelbar anschließenden aufweitenden Bereich kein Nachteil ist, wie man bisher annahm (DE 43 43 117). Vielmehr wird durch den sich aufweitenden Bereich des Schraubenkopfes, der durch das Durchgangsloch der Platte teilweise hindurch und in das Knochengewebe eintreten kann, zusätzlich eine größere Knochenschraube-Knochen-Kontaktfläche geschaffen. Die Lasten und Kräfte können somit besser verteilt übertragen werden.

Vorteilhafterweise ist die Gewindetiefe des Gewindes an dem aufweitenden Bereich geringer als die Gewindetiefe des Knochengewindes am Schaft.

Um eine wirksame Verblockung des Schraubenkopfes in der Lochwand einer Knochenplatte sicherzustellen, weist der Kopfbereich gemäß einer bevorzugten Ausführungsform der Erfindung ein mehrläufiges, beispielsweise ein doppelläufiges, Gewinde auf, wobei die Gewindesteigung beibehalten wird. Beim Eindrehen der Knochenschraube in den Knochen kann der in den Knochen eintauchende untere Kopfbereich mit seinem doppelläufigen Gewinde das bereits durch das Knochengewinde des Schaftes geschaffene Gewinde im Knochen ausnutzen. Das Knochengewinde der Schraube hat nämlich ein Gewinde in die Wand des Knochenloches geschnitten, so dass eine Flanke des doppelläufigen Gewindes des Kopfes hier einen direkten Kontakt mit dem Knochengewebe hat. Der zweite Gewindegang des doppelläufigen Gewindes muss sich allerdings selbständig in den Knochen hineinschneiden. Um dieses gut ausführen zu können, ist es vorteilhaft, dass die Gewindekanten des doppelläufigen Gewindes selbstschneidend sind. Zweckmäßigerweise kann das in den Knochen eintauchende Gewinde des Kopfes längliche Ausnehmungen aufweisen, um ggf. Knochenmaterial, welches beim Einschneidevorgang gebildet wird, aufnehmen zu können.

Gemäß einer bevorzugten Ausführungsform der Erfindung weitet sich der dem Schaft anschließende Kopfbereich der Knochenschraube in Bezug auf die Längsachse des Schaftes in einen Winkel von 12 bis 25 Grad, vorzugsweise 13,5 bis 15,5 Grad, weiter vorzugsweise 14,5 Grad auf und ist vorzugsweise ein Senkkopf oder Konus, Linsenkopf, Kugelkopf oder Birnenkopf.

Die Erfindung hat erkannt, dass innerhalb der vorgenannten Winkelbereiche, insbesondere innerhalb der bevorzugten Winkelbereiche, die eingangs beschriebene unerwünschte Spanbildung weitestgehend vermieden werden kann. Dies wird durch die Beispiele 2 bis 5 bestätigt. Es wird angenommen, dass die längliche Kopfform der Knochenschraube zu einer gleichmäßigeren und stärkeren Verpressung des umgeformten Materials in der Lochwand führt und somit Späne, die aus dem Loch heraustreten können, wirkungsvoll vermieden werden können.

Ein weiterer Vorteil der durch die bevorzugten Winkelbereiche vorgegebenen Kopfform ist die verbesserte Anbringung eines Werkzeugangriffs am Schraubenkopf, beispielsweise eines Torx- bzw. Sechskantloches zur Aufnahme eines geeigneten Schraubendrehwerkzeuges. Da sich der Schraubenkopf in Richtung des Schaftes nur langsam verengt, besteht die Möglichkeit, die Aufnahme für das Werkzeug, beispielsweise für einen Schraubendreher, tiefer und damit im Durchmesser kleiner zu gestalten, ohne dass es zu einer Schwächung der Wand im Kopfbereich kommt. Wenn der Kopf der Knochenschraube ein Konus ist, dann entspricht der Winkel des Konus der Aufnahme zweckmäßigerweise dem Winkel des Kopf-Konus. Hiermit bleibt die Wandstärke zwischen Aufnahme und Kopfaußenkontur unverändert. Vorteilhaft ist auch eine trompetenartige Gestaltung der Ausnehmung, wobei der Eingang des Werkzeugangriffs weiter ist und diese Weite sich zum Kopfinneren hin trompetenförmig verringert. Somit ist gewährleistet, dass die Kraftübertragung über eine größere Auflagefläche erzeugt und somit eine Zerstörung sowohl der Aufnahme im Schraubenkopf als auch im Bereich des Werkzeuges, z. B. der Klinge des Schraubendrehers, vermieden wird.

Gegenstand einer bevorzugten Ausführungsform der Erfindung ist des Weiteren ein Fixationssystem für Knochen mit einem Verbindungsträger mit wenigstens einem Durchgangsloch, in das die erfindungsgemäße Knochenschraube eingesetzt wird, wobei die Verbindung von Knochenschraube mit dem Verbindungsträger winkelstabil, vorzugsweise multidirektional winkelstabil, ist.

Weitere Einzelheiten und Vorteile der Erfindung ergeben sich aus der nachfolgenden Beschreibung der anliegenden Zeichnungen der bevorzugten Ausführungsformen und den Beispielen. Es zeigen:
- Fig. 1: eine erfindungsgemäße Knochenschraube in der Vorderansicht;
- Fig. 2: einen Querschnitt einer erfindungsgemäßen Knochenschraube;
- Fig. 3: einen Querschnitt von einer erfindungsgemäßen Knochenschraube in einem Knochen und verblockt in einem Plattenloch einer Knochenplatte;
- Fig. 4: einen Schraubenkopf von oben mit Sechskantinbus(a) bzw. Torx-Aufnahme (b);
- Fig. 5: eine in ein Loch mit Lochlippe eingeschraubte Knochenschraube;
- Fig. 6: das Loch gemäß Figur 5 nach der Entfernung der Knochenschraube;
- Fig. 7: eine weitere in ein Loch mit Lochlippe eingeschraubte Knochenschraube;
- Fig. 8: den Kopfteil der Knochenschraube gemäß Figur 6 nach der Entfernung aus dem Loch; und
- Fig. 9: das Loch gemäß Figur 7 nach der Entfernung der Knochenschraube; und
- Fig. 10: eine Bohrvorrichtung.

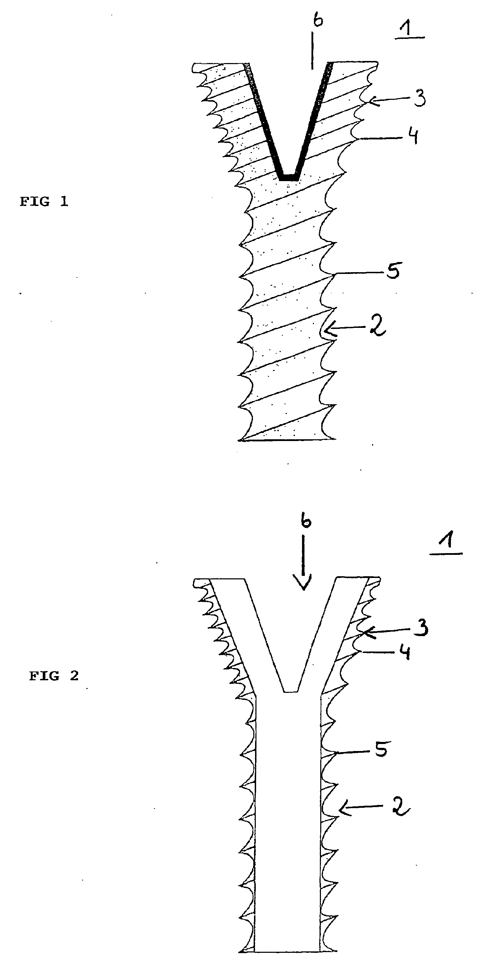

Die Figuren 1 und 2 zeigen eine erfindungsgemäße Knochenschraube 1 in der Vorderansicht und im Querschnitt. Die Knochenschraube 1 weist einen Schaft 2 mit einem Knochengewinde 5 und einen konusförmigen Kopf 3 mit einem Gewinde 4 auf. Das Knochengewinde 5 geht unterbrechungsfrei in das mehrläufige (doppelläufige) Gewinde 4 an dem aufweitenden Bereich des Kopfes 3 über, wobei die Gewindetiefe des Gewindes 4 geringer ist als die des Knochengewindes 5. Das Gewinde 4 weist die gleiche Gewindesteigung wie das Knochengewinde 4 auf. An der Oberseite des Kopfes 3 ist ein Werkzeugangriff 6 in Form einer konusförmigen Ausnahme angeordnet, in die ein entsprechendes Werkzeug eingreifen kann.

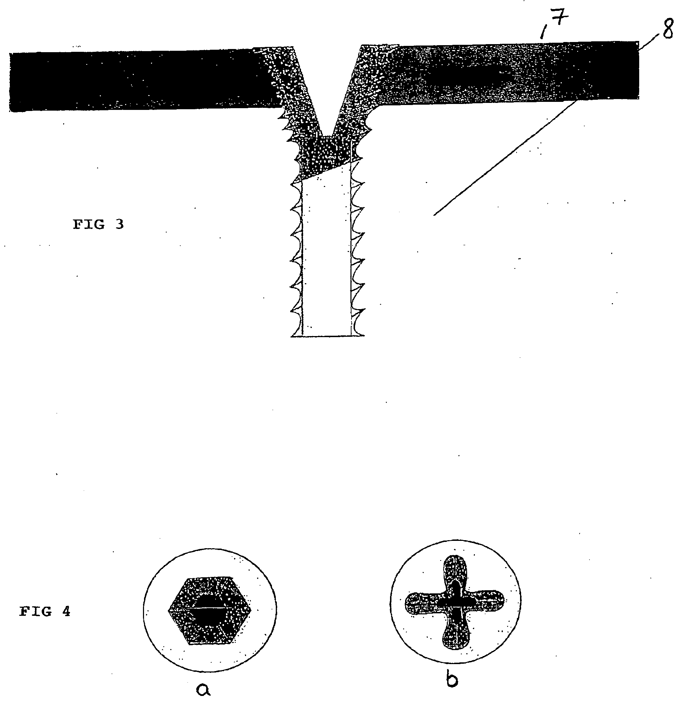

Figur 3 zeigt die in den Figuren 1 und 2 dargestellte Knochenschraube 1, die in einen Knochen 8 eingeschraubt und mit ihrem Kopf 3 in einem Plattenloch einer Knochenplatte 7 verblockt ist. Das durch den Kopf 3 umgeformte Material des Plattenloches hat sich in die Lochwand eingepresst und das Material an diesen Stellen verdichtet. Der sich den Schaft unmittelbar anschließende konusförmig erweiterte untere Kopfteil 3 taucht in das Knochengewebe 8 ein.

Ein Eindrehen bzw. Entfernen der Knochenschraube ist über einen in Fig. 4 dargestellten Werkzeugangriff in Form einer Aufnahme für einen Sechskantinbus (a) oder Torx (b) möglich.

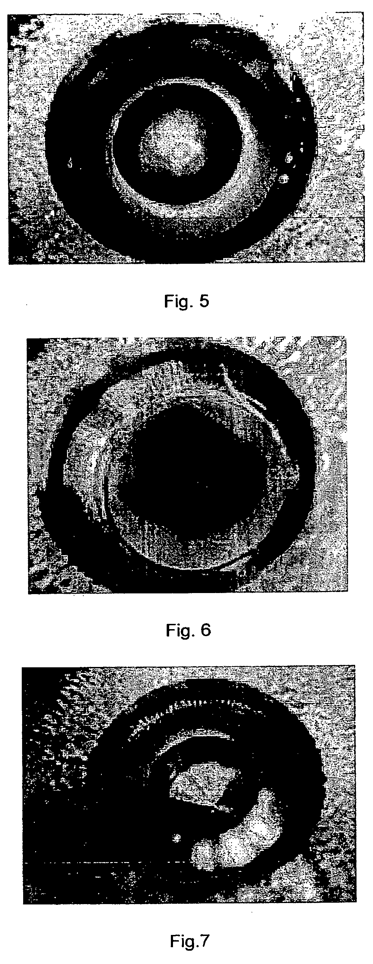

Figur 5 zeigt eine Knochenschraube 1 gemäß den Figuren 1 bis 3, die in einem Plattenloch mit Lippe verblockt ist. Aus dem Plattenloch steigt ein Span mit einer Länge von ca. 5 mm auf.

Figur 6 entspricht Figur 5 und zeigt das Plattenloch nach der Entfernung der Knochenschraube 1. Die Lippe ist eingefurcht. Ein ca. 5 mm langer Span ist abgeschert.

Figur 7 entspricht Figur 5, wobei eine erfindungemäße Knochenschraube mit einem 14,5° Kegelkopf verwendet wurde. Aus dem Plattenloch steigt kein Span auf.

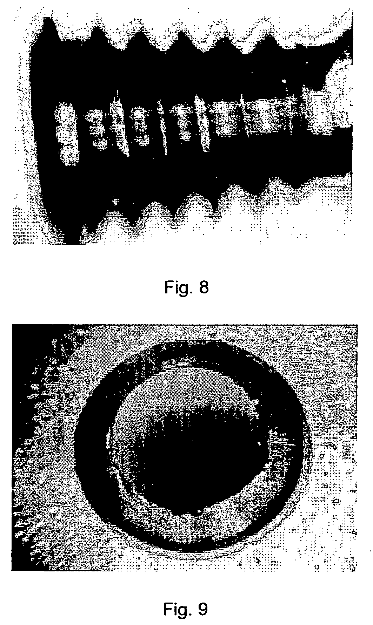

Figur 8 zeigt den Kopfteil der 14,5° Kegelkopf-Knochenschraube aus Figur 7 nach dem Entfernen aus dem Plattenloch.

Figur 9 entspricht Figur 7 und zeigt das Plattenloch in der Knochenplatte nach dem Entfernen der Knochenschraube. Es ist keine Spanbildung ersichtlich. Die Lippe ist nur leicht eingefurcht.

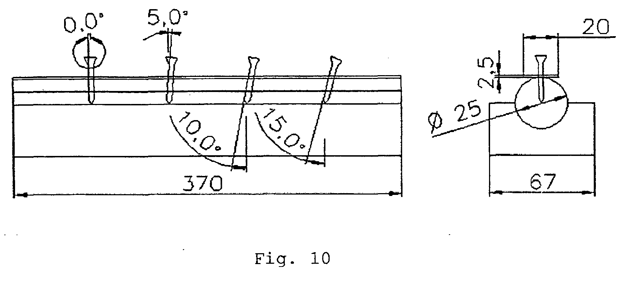

Figur 10 zeigt die Eindrehversuche in einer Bohrvorrichtung. Links ist die Bohrvorrichtung mit der flexiblen Schlittenschablone, mit der die verschiedenen Eindrehwinkel in den Kunstknochen gebohrt werden, dargestellt. Rechts ist gezeigt, wie die tifix^{®}MINI 1 Schraube in den Kunstknochen eingeschraubt und in der Lippe verblockt wird.

Zur Beurteilung der Spanbildung beim Eindrehen der erfindungsgemäßen Knochenschrauben in Abhängigkeit von dem Winkel des aufweitenden Bereiches des Kopfes wurden die in den nachfolgenden Beispielen beschriebenen Versuche durchgeführt. Im Einzelnen:

### Beispiel 1: Versuchsaufbau

Standartplatten Titan 1 mit 125x20x2,5 (LxBxH) a 12 Löcher. Eindrehen von Knochenschrauben (Typ tifix^{®} MINI 1 - Titan 4 (hochfestes Reintitan)) in Kunstknochen mit unterschiedlicher Kegelkopfgeometrie (14, 5°, 16°, 17,5°) sowie definierten Eindrehwinkeln (5°, 10°). Anzugsdrehmoment = 2,5-3 Nm.

### Beispiel 2:

Es wurden 17,5 ° Kegelkopf-Knochenschrauben bei einem Eindrehwinkel von 5° gemäß dem in Beispiel 1 beschriebenen Versuchsaufbau untersucht. In 3 von 5 Fällen war im verblockten Zustand eine Spanbildung ersichtlich.

### Beispiel 3:

Es wurden 16 ° Kegelkopf-Knochenschrauben bei einem Eindrehwinkel von 5° gemäß dem in Beispiel 1 beschriebenen Versuchsaufbau untersucht. In 3 von 5 Fällen war im verblockten Zustand eine Spanbildung ersichtlich.

### Beispiel 4:

Es wurden 16° Kegelkopf-Knochenschrauben bei einem Eindrehwinkel von 10° gemäß dem in Beispiel 1 beschriebenen Versuchsaufbau untersucht. In 1 von 5 Fällen war im verblockten Zustand eine Spanbildung ersichtlich.

### Beispiel 5 :

Es wurden 14,5 ° Kegelkopf-Knochenschrauben bei einem Eindrehwinkel von 5° gemäß dem in Beispiel 1 beschriebenen Versuchsaufbau untersucht. In keinem Fall war im verblockten Zustand eine Spanbildung ersichtlich.

Die Versuchsergebnisse der Beispiele 2 bis 5 zeigen, dass mit abnehmendem Kegelwinkel des Knochenschraubenkopfes weniger Späne aus dem Knochenloch aufsteigen. Keine Späne werden bei einem Kegelwinkel von 14,5 ° erzeugt, da offenbar über diese Geometrie deutlich geringere Kräfte erzeugt werden. Das führt dazu, dass weder am Schraubenkopf noch an dem Schraubenloch Material abschert und auch die Lippe deutlich weniger beschädigt wird.

## Patentansprüche

1. Fixationssystem für Knochen (8) umfassend
- einen Verbindungsträger (7), vorzugsweise eine Knochenplatte, mit mindestens einem Durchgangsloch, und
- eine Knochenschraube (1) mit einem eine Längsachse definierenden Schaft (2), der ein Knochengewinde (5) trägt, und einem als Verdickung ausgeführten Kopf (3) der ein Gewinde (4) trägt, wobei der Kopf (3) einen aufweitenden Bereich aufweist,
wobei das Knochengewinde (5) des Schafts (2) unterbrechungsfrei in das Gewinde (4) an dem aufweitenden Bereich der Verdickung (3) übergeht, und wobei bei der Fixierung einer Knochenplatte (7) an einem Knochen (8) mittels der Knochenschraube (1) der aufweitende Bereich des Kopfes (3) der Knochenschraube (1) durch ein Durchgangsloch der Knochenplatte (7) teilweise hindurch tritt und im Knochengewebe (8) zum Liegen kommt, **dadurch gekennzeichnet, dass** der aufweitende Bereich des Kopfes (3) in Bezug auf die Längsachse des Schaftes (2) in einem Winkel von 10 bis 28 Grad aufweitet, und das Gewinde (4) an dem aufweitenden Bereich des Kopfes (3) die Gewindesteigung des Knochengewindes (5) am Schaft (2) aufweist.

2. Fixationssystem nach Anspruch 1, **dadurch gekennzeichnet, dass** der aufweitende Bereich des Kopfes (3) ein mehrläufiges, vorzugsweise ein doppelläufiges Gewinde (4) aufweist.

3. Fixationssystem nach einem der vorangehenden Ansprüche, **dadurch gekennzeichnet, dass** das Gewinde (4) an dem aufweitenden Bereich des Kopfes (3) ein selbstschneidendes Gewinde ist.

4. Fixationssystem nach einem der vorangehenden Ansprüche, **dadurch gekennzeichnet, dass** die Gewindetiefe des Gewindes (4) an dem aufweitenden Bereich des Kopfes (3) geringer ist als die Gewindetiefe des Knochengewindes (5) am Schaft (2).

5. Fixationssystem nach einem der vorangehenden Ansprüche, **dadurch gekennzeichnet, dass** sich der aufweitende Bereich des Kopfes (3) in Bezug auf die Längsachse des Schaftes (2) in einem Winkel von 12 bis 25 Grad, vorzugsweise 13,5 bis 15,5 Grad, weiter vorzugsweise 14,5 Grad aufweitet.

6. Fixationssystem nach einem der vorangehenden Ansprüche, **dadurch gekennzeichnet, dass** sich der aus dem Schaft (2) erstreckende Kopf (3) ein Senkkopf oder Konus, Linsenkopf, Kugelkopf oder Birnenkopf ist.

7. Fixationssystem nach einem der vorangehenden Ansprüche, **dadurch gekennzeichnet, dass** der Kopf (3) einen Werkzeugangriff (6) aufweist.

8. Fixationssystem nach einem der vorangehenden Ansprüche, **dadurch gekennzeichnet, dass** der im Knochengewebe (8) liegende, aufweitende Bereich des Kopfes (3) Spanaufnahmebereiche aufweist.

9. Fixationssystem für Knochen (8) nach einem der vorangehenden Ansprüche, wobei in das Durchgangsloch eine Knochenschraube (1) nach einem der vorangehenden Ansprüche einsetzbar ist und die Verbindung von Knochenschraube (1) mit dem Verbindungsträger (7) winkelstabil, vorzugsweise multidirektional winkelstabil, ist.

## Claims

1. Fixation system for bones (8), comprising
- a connecting support (7), preferably a bone plate, with at least one through-hole, and
- a bone screw (1) with a shaft (2) that defines a longitudinal axis and has a bone thread (5), and with a head (3) that is configured as a thickened portion and that has a thread (4), wherein the head (3) has a flared area, wherein the bone thread (5) of the shaft (2) merges in an uninterrupted manner into the thread (4) on the flared area of the thickened portion (3) and, wherein, when a bone plate (7) is fixed to a bone (8) by means of the bone screw (1), part of the flared area of the head (3) of the bone screw (1) passes through a through-hole in the bone plate (7) and comes to lie in the osseous tissue (8), **characterized in that** the flared area of the head (3) widens, relative to the longitudinal axis of the shaft (2), at an angle of 10 to 28 degrees, and the thread (4) on the flared area of the head (3) has the thread pitch of the bone thread (5) on the shaft (2).

2. Fixation system according to Claim 1, **characterized in that** the flared area of the head (3) has a multi-run thread (4), preferably a double-run thread (4).

3. Fixation system according to either of the preceding claims, **characterized in that** the thread (4) on the flared area of the head (3) is a self-tapping thread.

4. Fixation system according to one of the preceding claims, **characterized in that** the thread depth of the thread (4) on the flared area of the head (3) is shallower than the thread depth of the bone thread (5) on the shaft (2).

5. Fixation system according to one of the preceding claims, **characterized in that** the flared area of the head (3) widens, relative to the longitudinal axis of the shaft (2), at an angle of 12 to 25 degrees, preferably of 13.5 to 15.5 degrees, more preferably of 14.5 degrees.

6. Fixation system according to one of the preceding claims, **characterized in that** the head (3) extending from the shaft (2) is a flat head or cone, a fillister head, a spherical head or a pear-shaped head.

7. Fixation system according to one of the preceding claims, **characterized in that** the head (3) has a tool engagement part (6).

8. Fixation system according to one of the preceding claims, **characterized in that** the flared area of the head (3) lying in the osseous tissue (8) has chip-receiving areas.

9. Fixation system for bones (8) according to one of the preceding claims, in which a bone screw (1) according to one of the preceding claims can be fitted into the through-hole, and the connection between bone screw (1) and connecting support (7) has angle stability, preferably multidirectional angle stability.

## Revendications

1. Système de fixation pour os (8) comprenant :
- un support de liaison (7), de préférence une plaque osseuse, avec au moins un trou de passage et
- une vis à os (1) avec une tige (2) définissant un axe longitudinal, qui présente un filetage à os (5), et une tête (3) réalisée sous la forme d'un épaississement, qui présente un filetage (4), la tête (3) comprenant une zone qui s'élargit, le filetage à os (5) de la tige (2) passant, sans interruption, dans le filetage (4) sur la zone qui s'élargit de l'épaississement (3) et, lors de la fixation d'une plaque osseuse (7) sur un os (8), au moyen de la vis à os (1) de la zone qui s'élargit de la tête (3) de la vis à os (1) traversant partiellement un trou de passage de la plaque osseuse (7) et entre en contact avec le tissu osseux (8), **caractérisé en ce que** la zone qui s'élargit de la tête (3) s'élargit par rapport à l'axe longitudinal de la tige (2) avec un angle de 10 à 28 degrés et le filetage (4) sur la zone qui s'élargit de la tête (3) comprend le pas du filetage à os (5) sur la tige (2).

2. Système de fixation selon la revendication 1, **caractérisé en ce que** la zone qui s'élargit de la tête (3) comprend un filetage (4) multiple, de préférence un filetage (4) double.

3. Système de fixation selon l'une des revendications précédentes, **caractérisé en ce que** le filetage (4) sur la zone qui s'élargit de la tête (3) est un filetage auto-taraudeur.

4. Système de fixation selon l'une des revendications précédentes, **caractérisé en ce que** la profondeur du filetage (4) sur la zone qui s'élargit de la tête (3) est inférieure à la profondeur du filetage à os (5) sur la toge (2).

5. Système de fixation selon l'une des revendications précédentes, **caractérisé en ce que** la zone qui s'élargit de la tête (3) s'élargit par rapport à l'axe longitudinal de la tige (2) avec un angle de 12 à 25 degrés, de préférence de 13,5 à 15,5 degrés, de préférence de 14,5 degrés.

6. Système de fixation selon l'une des revendications précédentes, **caractérisé en ce que** la tête (3) s'étendant à partir de la tige (2) est une tête fraisée ou un cône, une tête bombée, une tête sphérique ou une tête en forme de poire.

7. Système de fixation selon l'une des revendications précédentes, **caractérisé en ce que** la tête (3) comprend une prise d'outil (6).

8. Système de fixation selon l'une des revendications précédentes, **caractérisé en ce que** la zone qui s'élargit de la tête (3), qui se trouve dans le tissu osseux (8), comprend des zones de logement de copeaux.

9. Système de fixation pour os (8) selon l'une des revendications précédentes, une vis à os (1) selon l'une des revendications précédentes pouvant être inséré dans le trou de passage et la liaison entre la vis à os (1) et le support de liaison (7) est stable angulairement, de préférence stable angulairement de manière multidirectionnelle.
